# EUROPEAN PATENT APPLICATION

(11) **EP 4 653 550 A1**
(43) Date of publication of application: **26.11.2025**
(21) Application number: 23917896.5
(22) Date of filing: 07.12.2023
(51) Int. Cl.: C12Q 1/70, C12Q 1/6825, C12Q 1/686

(54) **METHOD AND CARTRIDGE FOR DIAGNOSING FOOT-AND-MOUTH DISEASE VIRUS USING SEMICONDUCTOR BIOSENSOR-BASED POLYMERASE CHAIN REACTION TECHNIQUE**

(30) Priority: 19.01.2023 KR 20230008053
(71) Applicant: Optolane Technologies Inc., Bundang-gu Seongnam-si, Gyeonggi-do 13494 (KR); REPUBLIC OF KOREA (ANIMAL AND PLANT QUARANTINE AGENCY), Gimcheon-si, Gyeongsangbuk-do 39660 (KR)
(72) Inventor: SHIN, Seung Shick, Gwangju-si Gyeonggi-do 12793 (KR); SEO, Seong Min, Hwaseong-si Gyeonggi-do 18482 (KR); LEE, Do Young, Seoul 06002 (KR); CHA, Sang Ho, Cheonan-si Chungcheongnam-do 31156 (KR); RYOO, So Yoon, Gimcheon-si Gyeongsangbuk-do 39660 (KR); KANG, Heon Jeong, Gimcheon-si Gyeongsangbuk-do 39660 (KR); LIM, Da Rae, Gumi-si Gyeongsangbuk-do 39290 (KR); KIM, Jea Myung, Gimcheon-si Gyeongsangbuk-do 39660 (KR)
(74) Representative: Boult Wade Tennant LLP
(86) International application number: PCT/KR2023/020059
(87) International publication number: WO 2024/154944

(57) **Abstract**

The present invention relates to a method for diagnosing foot-and-mouth disease virus (FMDV) using polymerase chain reaction (PCR), and more particularly, to a method for diagnosing foot-and-mouth disease virus that enables highly accurate and multiplex diagnosis of foot-and-mouth disease virus using a semiconductor biosensor-based PCR technique. According to the present invention, the method can satisfy both the simplicity and accuracy of early diagnosis of foot-and-mouth disease, thereby playing an important role in preventing the spread of foot-and-mouth disease virus through early diagnosis and management. In addition, the present invention has high potential in meeting the demand for on-site early diagnosis and for diagnostic kits for foot-and-mouth disease.

## Description

### [Technical Field]

The present invention relates to a method for diagnosing foot-and-mouth disease virus (FMDV) using polymerase chain reaction (PCR), and more particularly, to a method for diagnosing foot-and-mouth disease virus that enables highly accurate and multiplex diagnosis of foot-and-mouth disease virus using a semiconductor biosensor PCR technique.

### [Background Art]

Foot-and-mouth disease infects cloven-hoofed animals such as cattle, pigs, sheep, goats, and deer. It corresponds to a Type 1 livestock infectious disease under the Livestock Infectious Disease Prevention Act and is designated as a major livestock infectious disease by the World Organisation for Animal Health (WOAH). Foot-and-mouth disease is highly contagious and has a very short incubation period of about 2 to 14 days. The foot-and-mouth disease virus, which causes foot-and-mouth disease, is an RNA virus composed of approximately 8.4 kb of single-stranded positive sense NA, +ssRNA). This virus is non-enveloped and consists of structural proteins VP1, VP2, VP3, and VP4, and several types of non-structural proteins.

Foot-and-mouth disease virus is classified into seven serotypes (A, O, Asia1, SAT1, SAT2, SAT3, and C), and these major serotypes are further classified into approximately 80 subtypes. In the Republic of Korea, the serotypes that predominantly occur are type O and type A. Since the seven serotypes of foot-and-mouth disease virus are clearly distinguishable and do not provide cross-protection between serotypes, rapid serotyping along with determination of positivity is required for the appropriate selection of vaccines.

In the past, most farms administered foot-and-mouth disease vaccines to prevent the occurrence and spread of the disease. Accordingly, even when infection with the foot-and-mouth disease virus occurs, animals often exhibit only mild symptoms such as vesicle formation or salivation, resulting in frequent delays in reporting suspected animals at the early stage of infection.

In general, the final confirmatory diagnosis of foot-and-mouth disease is conducted by sending test samples such as vesicular fluid, vesicular epithelial cells, and serum to the Foot-and-Mouth Disease Diagnosis Division of the Animal and Plant Quarantine Agency or to provincial foot-and-mouth disease diagnostic institutions designated by the Animal and Plant Quarantine Agency. That is, when a suspected case of foot-and-mouth disease is reported, a livestock quarantine officer is first dispatched to the farm to observe clinical symptoms, and if foot-and-mouth disease is suspected, samples are collected. Moreover, if testable samples (such as vesicles or scabs) are available, a rapid antigen diagnostic kit is used on-site to confirm the result. The collected samples are then transported to a laboratory, where antibody testing, antigen testing, and genetic testing are conducted to accurately determine whether the sample is positive, and if positive, to identify the virus type.

However, since this process takes 2 to 3 days from the report of a suspected case of foot-and-mouth disease to diagnosis and confirmation due to the transportation and testing of samples, additional measures, such as restricting the movement of livestock, are required to prevent the possibility of further spread while waiting for the confirmation of positivity and virus type. Furthermore, livestock vaccinated against foot-and-mouth disease often produce a low level of virus upon infection with the foot-and-mouth disease virus, which may result in false-negative diagnoses due to the limited sensitivity of rapid diagnostic kits, thereby making accurate determination difficult in many cases.

To address the issue of time delay, interest in implementing on-site testing is increasing, and conventionally, studies have been conducted on methods such as lateral flow assay and loop-mediated isothermal amplification (LAMP). However, these methods have the drawback of low sensitivity, which may lead to false-negative diagnoses, and have the limitation that only a single type of marker can be detected per kit.

Therefore, there is a need for a method capable of accurately diagnosing foot-and-mouth disease at an early stage through a sensitive and rapid on-site diagnostic test.

### [Detailed Description of the Invention]

### [Technical Problem]

The present invention relates to a method for diagnosing foot-and-mouth disease virus (FMDV) using polymerase chain reaction (PCR) and is intended to provide a method for diagnosing foot-and-mouth disease virus that enables highly accurate and multiplex diagnosis of foot-and-mouth disease virus using a semiconductor biosensor PCR technique.

### [Technical Solution]

To achieve the above problem, one aspect of the present invention provides a method for diagnosing foot-and-mouth disease virus, the method comprising:
a liquid sample injection step of injecting a liquid sample into an inlet to fill a plurality of wells;
a sensor contact step of bringing the plurality of wells filled with the liquid sample into close contact with a CMOS photosensor;
a fluorescence signal detection step of detecting a fluorescence signal through the CMOS photosensor;
a Ct calculation step of calculating one or more cycle threshold (Ct) values for each of the plurality of wells from the detected fluorescence signals; and
a diagnosis step of diagnosing the presence and type of virus based on the cycle threshold values,
wherein the method diagnoses one or more of foot-and-mouth disease type O, foot-and-mouth disease type A, foot-and-mouth disease type Asia1, foot-and-mouth disease type O/ME-SA/Ind-2001, foot-and-mouth disease type O/ME-SA/PanAsia, foot-and-mouth disease type O/SEA/Mya-98, foot-and-mouth disease type O/CATHAY, foot-and-mouth disease type A/ASIA/Sea-97, foot-and-mouth disease type A/ASIA/G-VII, vesicular stomatitis virus (VSV), swine vesicular disease virus (SVDV), and seneca valley virus (SVV).

According to one embodiment, in the injection step, the plurality of wells may each independently include primers and probes targeting one or more of foot-and-mouth disease type O, foot-and-mouth disease type A, foot-and-mouth disease type Asia1, foot-and-mouth disease type O/ME-SA/Ind-2001, foot-and-mouth disease type O/ME-SA/PanAsia, foot-and-mouth disease type O/SEA/Mya-98, foot-and-mouth disease type O/CATHAY, foot-and-mouth disease type A/ASIA/Sea-97, foot-and-mouth disease type A/ASIA/G-VII, vesicular stomatitis virus (VSV), swine vesicular disease virus (SVDV), and seneca valley virus (SVV).

According to one embodiment, in the injection step, the plurality of wells may include a first fluorescent substance or a second fluorescent substance.

According to one embodiment, in the injection step, the first fluorescent substance may be bound to a first probe targeting one of foot-and-mouth disease type O, foot-and-mouth disease type A, foot-and-mouth disease type Asia1, foot-and-mouth disease type O/ME-SA/Ind-2001, foot-and-mouth disease type O/ME-SA/PanAsia, foot-and-mouth disease type O/SEA/Mya-98, foot-and-mouth disease type O/CATHAY, foot-and-mouth disease type A/ASIA/Sea-97, foot-and-mouth disease type A/ASIA/G-VII, and foot-and-mouth disease-like viruses including vesicular stomatitis virus (VSV), swine vesicular disease virus (SVDV), and seneca valley virus (SVV), and may be applied to one of the plurality of wells, and the second fluorescent substance may be bound to a second probe targeting one of foot-and-mouth disease type O, foot-and-mouth disease type A, foot-and-mouth disease type Asia1, foot-and-mouth disease type O/ME-SA/Ind-2001, foot-and-mouth disease type O/ME-SA/PanAsia, foot-and-mouth disease type O/SEA/Mya-98, foot-and-mouth disease type O/CATHAY, foot-and-mouth disease type A/ASIA/Sea-97, foot-and-mouth disease type A/ASIA/G-VII, and foot-and-mouth disease-like viruses including vesicular stomatitis virus (VSV), swine vesicular disease virus (SVDV), and seneca valley virus (SVV), and may be applied to one of the plurality of wells.

According to one embodiment, in the injection step, at least one first probe and at least one second probe may be applied together to one of the plurality of wells.

According to another aspect of the present invention, there is provided a PCR analysis method using a PCR cartridge, the cartridge comprising: a microfluidic chamber including an inlet formed for introduction of a liquid sample and capable of being produced by injection molding; a well array including a plurality of microwells through which upper and lower portions are perforated, the well array being attached to a lower surface of the microfluidic chamber; a CMOS photosensor array disposed below the well array and configured to capture real-time reaction images of samples filled in the microwells of the well array; and
a PCB having a vent formed for vacuum processing of a microchannel that is provided in the microfluidic chamber, a space that is created between the well array and the microfluidic chamber, and the microwells that is formed in the well array, as the liquid sample is introduced through the inlet,
the method comprising:
   a liquid sample injection step of injecting the liquid sample into the inlet to fill the microwells;
   a sensor contact step of bringing the microwells filled with the liquid sample into close contact with a CMOS photosensor;
   a raw image acquisition step of acquiring raw images through the CMOS photosensor;
   a fractionated image acquisition step of acquiring a plurality of fractionated images corresponding to each of the plurality of microwells facing the CMOS photosensor from the raw images;
   a color parameter extraction step of extracting color parameters for each of a plurality of pixels assigned to each of the plurality of fractionated images;
   a fraction determination value calculation step of calculating fraction determination values for each of the plurality of fractionated images based on an average value of the color parameters for the plurality of pixels; and
   a final calculation step of calculating a final result value based on the plurality of fraction determination values,
   wherein, in the raw image acquisition step, the raw image is acquired at each predetermined time point, and
   wherein the PCR analysis method diagnoses one or more of foot-and-mouth disease type O, foot-and-mouth disease type A, foot-and-mouth disease type Asia1, foot-and-mouth disease type O/ME-SA/Ind-2001, foot-and-mouth disease type O/ME-SA/PanAsia, foot-and-mouth disease type O/SEA/Mya-98, foot-and-mouth disease type O/CATHAY, foot-and-mouth disease type A/ASIA/Sea-97, foot-and-mouth disease type A/ASIA/G-VII, vesicular stomatitis virus (VSV), swine vesicular disease virus (SVDV), and seneca valley virus (SVV).

According to one embodiment, the fractionated image acquisition step, the color parameter extraction step, the fraction determination value calculation step, and the final result value calculation step may be performed with respect to each raw image acquired at each predetermined time point, and the fraction determination value and the final result value may be obtained as time-series data.

According to one embodiment, the raw image acquisition step may be performed after confirming that the plurality of microwells have been filled with the liquid sample.

According to one embodiment, the fractionated image acquisition step may include: distinguishing the walls of the plurality of microwells from the plurality of microwells on the raw image; and acquiring, as the fractionated image, a portion of the raw image corresponding to each of the plurality of microwells.

According to one embodiment, in the color parameter extraction step, the color parameter may include at least one of brightness, saturation, hue, contrast, and color code.

According to one embodiment, in the final result value calculation step, the degree of wetting of each of the plurality of microwells with respect to the liquid sample may be determined, and the final result value may be calculated only from the fraction determination values for the fractionated images of the microwells having a degree of wetting equal to or higher than a predetermined level.

According to one embodiment, in the final result value calculation step, the operational status of each of the plurality of microwells may be determined, and the final result value may be calculated only from the fraction determination values for the fractionated images of the microwells corresponding to a normal state among the plurality of microwells.

According to still another aspect of the present invention, there is provided a foot-and-mouth disease virus test system for performing foot-and-mouth disease virus diagnosis by the method described above, the system comprising:
a foot-and-mouth disease test cartridge including an in vitro diagnostic chip therein;
a foot-and-mouth disease test analysis module in which the foot-and-mouth disease test cartridge is mounted, the foot-and-mouth disease test analysis module being configured to receive an image or fluorescence signal from the foot-and-mouth disease test cartridge and to calculate a fraction determination value or a Ct value; and
a main server configured to receive the fraction determination value via a communication unit of the foot-and-mouth disease test analysis module.

According to one embodiment, the foot-and-mouth disease test analysis module may include a battery that supplies power and a communication unit that is communicatively connected to the main server.

Other specific details of the embodiments according to the present invention are included in the following detailed description.

### [Advantageous Effects]

According to the method for diagnosing foot-and-mouth disease virus of the present invention, multiple types of viruses can be simultaneously detected by a single test, thereby minimizing the time and cost required for diagnosis. Moreover, foot-and-mouth disease can be diagnosed at an early stage on-site with high sensitivity and accuracy. Compared to conventional temporary rapid diagnostic kits used on-site, the method provides higher sensitivity and satisfies both convenience and accuracy in diagnosis. Accordingly, it can play an important role in preventing the initial spread of foot-and-mouth disease virus through early detection.

According to one embodiment, the method for diagnosing foot-and-mouth disease virus of the present invention performs diagnosis in a plurality of wells to which primers targeting different viruses are independently applied, thereby determining the presence of infection with multiple types of viruses at once by a single test.

According to another embodiment, the method for diagnosing foot-and-mouth disease virus of the present invention can be implemented as a digital real-time PCR method that provides a real-time graph for each individual fraction or individual well, thereby eliminating false-positive or false-negative errors. Furthermore, the present invention can prevent the formation of air pockets at the corner or edge regions of the well array, which serves as the reaction space. Accordingly, it is possible to prevent errors in PCR test results caused by such air pockets. In addition, since the well array is positioned on top of the CMOS photosensor array, the real-time PCR reaction can be measured.

### [Brief Description of Drawings]

FIG. 1 is a perspective view schematically illustrating a PCR cartridge according to an embodiment of the present invention.
FIG. 2 is an exploded perspective view of the PCR cartridge illustrated in FIG. 1.
FIG. 3A is a perspective view schematically illustrating the shape of a well array of a PCR cartridge according to one embodiment.
FIG. 3B is a perspective view schematically illustrating the shape of a well array of a PCR cartridge according to another embodiment.
FIG. 4A is a diagram showing the results of multiplex real-time PCR.
FIGS. 4B and 4C are diagrams showing the results of digital real-time PCR.
FIG. 5A is a front perspective view schematically illustrating the microfluidic chamber illustrated in FIG. 2, FIG. 5B is a rear perspective view schematically illustrating the microfluidic chamber illustrated in FIG. 2, and FIG. 5C is an exploded perspective view of the microfluidic chamber illustrated in FIG. 2.
FIG. 6 is a cross-sectional view schematically illustrating the PCR module illustrated in FIG. 2.
FIG. 7 is an exploded cross-sectional view schematically illustrating the PCR module illustrated in FIG. 6.
FIG. 8 is a cross-sectional view schematically illustrating a first step of the PCR process using the PCR module illustrated in FIG. 6.
FIG. 9 is a cross-sectional view schematically illustrating a second step of the PCR process using the PCR module illustrated in FIG. 6.
FIGS. 10, 11, 12 and 13 are cross-sectional views schematically illustrating a third step of the PCR process using the PCR module illustrated in FIG. 6.
FIGS. 14, 15 and 16 are cross-sectional views schematically illustrating a fourth step of the PCR process using the PCR module illustrated in FIG. 6.
FIGS. 17, 18, 19, 20 and 21 are cross-sectional views schematically illustrating a fifth step of the PCR process using the PCR module illustrated in FIG. 6.
FIG. 22 is a schematic diagram of a method for diagnosing foot-and-mouth disease virus according to one embodiment.
FIG. 23 shows graphs illustrating the respective virus titers (TCID₅₀) according to Example 1.
FIG. 24 shows graphs illustrating the results according to Experimental Example 1.

### [Mode for Carrying Out the Invention]

While the present invention is susceptible to various modifications and alternative forms, specific embodiments are illustrated in the drawings and described in detail herein. However, it is to be understood that the invention is not limited to the specific embodiments, but encompasses all modifications, equivalents, and alternatives falling within the spirit and scope of the invention.

Like reference numerals are used to denote like elements in describing each drawing. In the accompanying drawings, the dimensions of structures are exaggerated for clarity of illustration.

Although the terms "first", "second", or the like may be used herein to describe various components, these components should not be limited by these terms. These terms are only used to distinguish one element from another. For example, without departing from the scope of the present invention, a first component may be referred to as a second component, and similarly, the second component may also be referred to as the first component. Singular forms are intended to include plural forms as well, unless the context clearly indicates otherwise.

It should be understood that, as used herein, the terms "comprise" or "have" are intended to specify the presence of stated features, numerals, steps, operations, components, parts, or combinations thereof, but do not preclude the presence or addition of one or more other features, numerals, steps, operations, components, parts, or combinations thereof.

In addition, unless otherwise defined, all terms used herein, including technical and scientific terms, have the same meaning as commonly understood by those skilled in the art to which the present invention pertains. Terms, such as those defined in commonly used dictionaries, should be interpreted as having a meaning that is consistent with their meaning in the context of the relevant art, and will not be interpreted in an idealized or overly formal sense unless explicitly defined otherwise.

Hereinafter, preferred embodiments of the present invention will be described in more detail with reference to the accompanying drawings. In the drawings, the same reference numerals are used to denote the same components, and repeated descriptions of the same components will be omitted.

The present invention aims to solve the problem that it takes 2 to 3 days from a suspected report of highly contagious foot-and-mouth disease to a definite diagnosis, and the problem that false-negatives occur due to the low sensitivity of temporary rapid diagnostic kits used for screening. The present invention provides a method for diagnosing foot-and-mouth disease virus by performing polymerase chain reaction (PCR), which has higher sensitivity than conventional temporary rapid diagnostic kits, based on a semiconductor biosensor, such as a complementary metal-oxide semiconductor (CMOS), so that it can be readily used on-site like the rapid diagnostic kits.

In conventional digital PCR technology, false-negative or false-positive errors may occur when the fluorescence intensity in each fraction lies between the levels for positive and negative, making it difficult to clearly determine whether the result is positive and negative. To address such problems, the present invention provides a digital real-time or multiplex real-time PCR method using a semiconductor biosensor. According to the present invention, the presence of foot-and-mouth disease virus can be rapidly diagnosed while simultaneously analyzing real-time determination values and virus titer (TCID₅₀) curves. That is, the PCR cartridge used in the method for diagnosing foot-and-mouth disease virus of the present invention may be optimized for digital real-time PCR diagnosis or multiplex real-time PCR diagnosis, as described below.

In digital PCR technology, false-negative or false-positive errors may occur when the fluorescence intensity in each fraction lies between the levels for positive and negative, making it difficult to clearly determine whether the result is positive and negative. To address such problems, the present invention provides a PCR method using a semiconductor biosensor. According to the present invention, the presence of foot-and-mouth disease virus can be rapidly diagnosed while simultaneously analyzing real-time determination values and virus titer (TCID₅₀) curves.

The present invention can simultaneously detect the presence of viruses related to foot-and-mouth disease virus in order to accurately diagnose the presence and type of foot-and-mouth disease virus. According to the present invention, multiple targets such as serotypes, genotypes, and related viruses can be comprehensively distinguished by a single test.

The present invention relates to a structure for loading samples, sample volumes, or reaction volumes in a predetermined arrangement on a substrate, and more specifically, to a structure for loading samples in a predetermined arrangement at respective reaction sites within the substrate.

In various embodiments, the devices, instruments, systems, and methods are provided for loading samples into an article used to detect targets in a large number of small volume samples. These targets may be any suitable biological targets, including, but are not limited to, DNA sequences (including cell-free DNA), RNA sequences, genes, oligonucleotides, molecules, proteins, biomarkers, cells (e.g., circulating tumor cells), or other suitable target biomolecules. In various embodiments, such biological components may be used in conjunction with various PCR, qPCR, and/or dPCR methods and systems in applications such as fetal diagnostics, multiplex dPCR, viral detection and quantification standards, genotyping, sequencing validation, mutation detection, detection of genetically modified organisms, rare allele detection, and copy number variation.

While generally applicable to quantitative polymerase chain reactions (qPCR) where a large number of samples are being processed, it should be recognized that any suitable PCR method may be used in accordance with various exemplary embodiments described herein. Suitable PCR methods include, but are not limited to, digital PCR, allele-specific PCR, asymmetric PCR, ligation-mediated PCR, multiplex PCR, nested PCR, qPCR, cast PCR, genome walking, and bridge PCR, for example.

As described below, according to various embodiments described herein, reaction sites may include, but are not limited to, through-holes, sample retainment regions, wells, indentations, spots, cavities, and reaction chambers, for example.

Various embodiments described herein are particularly suited for digital PCR (dPCR). In digital PCR, a solution containing a relatively small number of a target polynucleotide or nucleotide sequence may be subdivided into a large number of small test samples, such that each sample generally contains either one molecule of the target nucleotide sequence or none of the target nucleotide sequence. Subsequently, when the samples are thermally cycled in a PCR protocol, procedure, or experiment, the samples containing the target nucleotide sequence are amplified and produce a positive detection signal, while the samples containing no target nucleotide sequence are not amplified and produce no detection signal. Using Poisson statistics, the number of target nucleotide sequences in the original solution may be correlated to the number of samples producing a positive detection signal.

In a cost-effective and efficient way to perform dPCR protocols, procedures, or experiments, it is advantageous to be able to divide an initial sample solution into tens of thousands or hundreds of thousands of volume areas, each having a volume of several nanoliters, at or about one nanoliter, or less than one nanoliter. Because the number of target nucleotide sequences may be very small, it may also be important in such circumstances that the entire content of the initial solution be accounted for and contained in the plurality of reaction sites.

Embodiments described herein solve these and other dPCR design constraints by distributing the initial sample solution into a plurality of reaction sites in a way that accounts for all, or essentially all, of sample solution.

For high throughput PCR assays and dPCR methods, a strategy of using an array format to reduce reaction volumes of liquid sample while increasing the number of reactions performed at one time may be employed. The array of reaction volumes of liquid sample may be in a substrate in a plurality of reaction sites. The reaction sites may be, but are not limited to, through-holes, wells, indentations, spots, cavities, reaction chambers, or any structure that may hold a sample, according to various embodiments described herein. In some embodiments, the through-holes or wells may be formed in a tapered shape having different diameters along the height.

Reduction in reaction volumes of liquid sample may allow for a higher density of reaction volumes so that more reactions can be performed within a given area. For example, an array of reaction sites comprised of 300 µm diameter through-holes in a substrate may contain about 30 nL of reaction volume. For example, by reducing the size of each through-hole in the array to 60 to 70 µm in diameter, each reaction volume may be 100 pL of liquid sample. Reaction volumes according to various embodiments described herein may range from about 1 pL to 30 nL of liquid sample. Furthermore, dynamic range may be increased by using more than one dilution of the liquid sample.

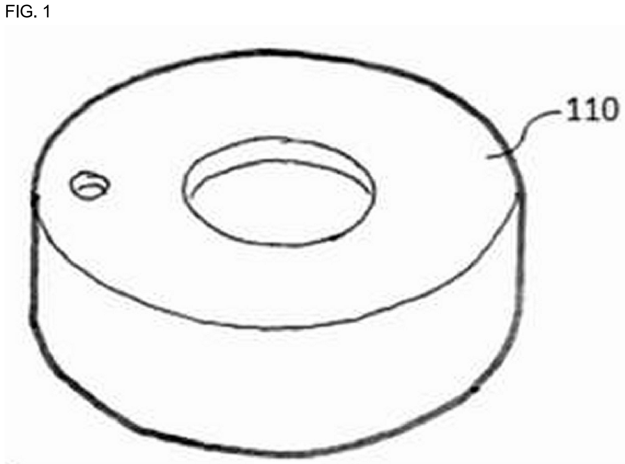

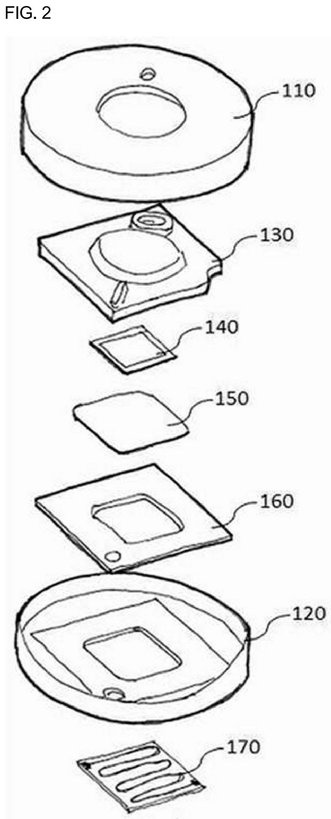

FIG. 1 is a perspective view schematically illustrating a PCR cartridge according to an embodiment of the present invention. FIG. 2 is an exploded perspective view of the PCR cartridge illustrated in FIG. 1.

Referring to FIGS. 1 and 2, a PCR cartridge according to an embodiment of the present invention is a cartridge-type test module and includes an upper case 110, a bottom case 120, a microfluidic chamber 130, a well array 140, 140', a CMOS photosensor array 150, and a PCB 160. In this embodiment, the PCR cartridge may be detachably coupled to a reader system of PCR equipment. In this embodiment, the microfluidic chamber 130, the well array 140, 140', the CMOS image sensor 150, and the PCB 160 may define a PCR module.

The upper case 110 includes a cover body of a donut shape including an upper hole formed in a central region and a window member disposed in the upper hole. The upper case 110 is coupled to the bottom case 120. In this embodiment, the upper case 110 and the bottom case 120 have a flat cylindrical shape, but various shapes may be possible. The window member may include a transparent material. The window member may include a PDMS material.

The bottom case 120 accommodates the microfluidic chamber 130, the well array 140, 140', the CMOS photosensor array 150, and the PCB 160, and is coupled to the upper case 110. The bottom case 120 and the upper case 110 may be coupled in a hook manner.

The microfluidic chamber 130 includes a membrane switch protruding upward and an inlet formed on one side of the membrane switch for injecting a liquid sample. A lower edge region of the microfluidic chamber 130 is in contact with the edge region of the PCB 160. A recessed space is formed in a lower central region of the microfluidic chamber 130 to accommodate a CMOS photosensor array 150 and the well array 140, 140' mounted on the PCB 160. The microfluidic chamber 130 may be formed of a material such as PDMS. The microfluidic chamber 130 has flexibility, transparency, PCR compatibility, and low autofluorescence, and can be produced by injection molding.

The well array 140, 140' is attached to a lower surface of the microfluidic chamber 130 and is disposed over the CMOS photosensor array 150. The well array 140, 140' may be composed of an etched silicon material.

The well array 140, 140' includes a plurality of wells that serve as PCR reaction sites. Each of the plurality of wells may have various shapes such as a hexagonal shape, a square shape, and the like.

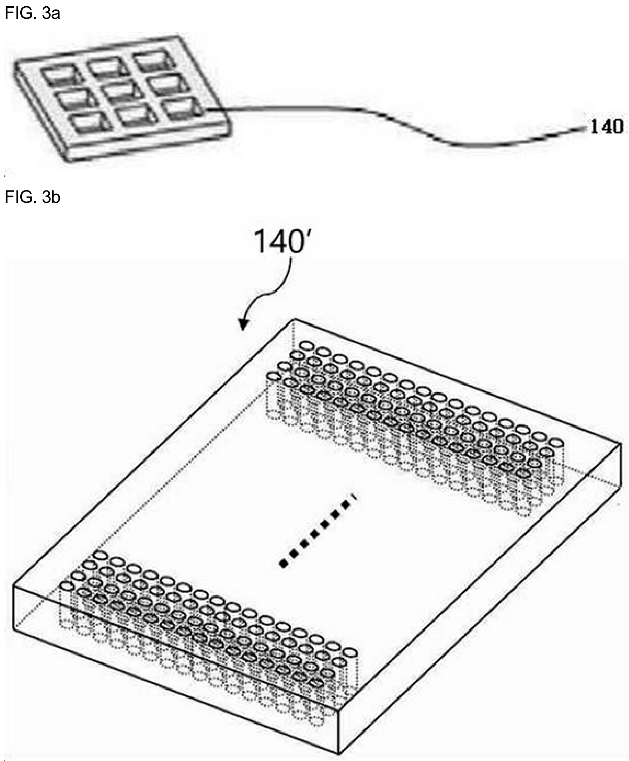

Referring to FIG. 3A or FIG. 3B, the shape, size, and volume of the plurality of wells may be adjusted. The plurality of wells have a shape in which the upper and lower portions penetrate. In this embodiment, the plurality of wells may have a thickness of less than 700 µm and a pitch of less than 150 µm or a pitch of less than 2500 µm. The plurality of wells may have various shapes such as a hexagonal shape, a circular shape, and the like. A hydrophilic coating layer may be formed on the well array 140, 140'. The well array 140, 140' may be attached to the microfluidic chamber 130 by plasma, thermal or UV curable adhesives.

As shown in FIGS. 3A and 3B, the specifications and number of the plurality of wells included in the well array 140, 140' may vary depending on the purpose and target of the analysis.

As illustrated in FIG. 3A, the plurality of wells of the well array 140 according to one embodiment may be provided in a number ranging from several to several tens. This is intended to enable the simultaneous detection of multiple types of viruses, and each of the plurality of wells forming the well array 140 may accommodate different primers and probes. In other words, each of the plurality of wells may target a different virus.

As illustrated in FIG. 3B, the plurality of wells of the well array 140' according to another embodiment may be microwells. Each of the microwells may be formed on a micrometer scale, and the well array 140' may include from thousands to hundreds of thousands of microwells. In this case, all of the microwells may target a single type of virus, and a real-time graph may be output for each well, thereby eliminating false-positive or false-negative results.

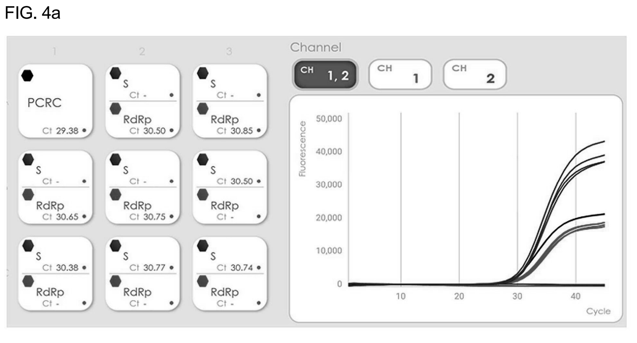

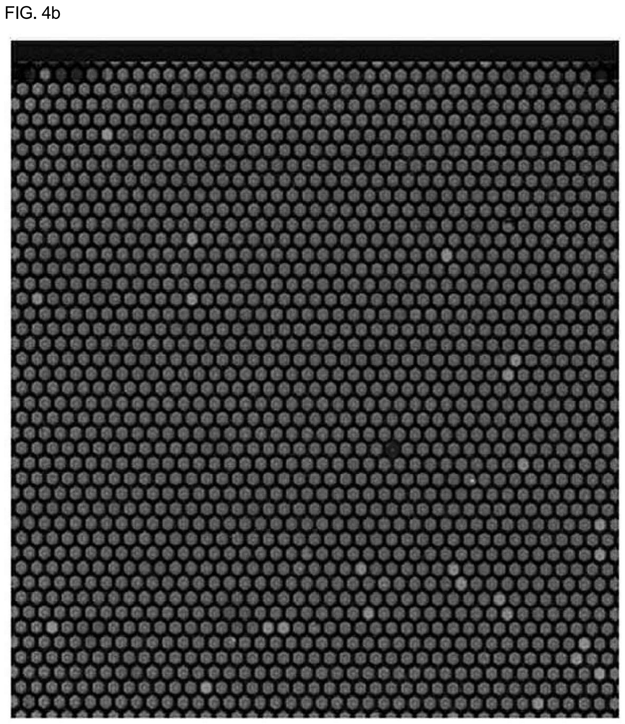

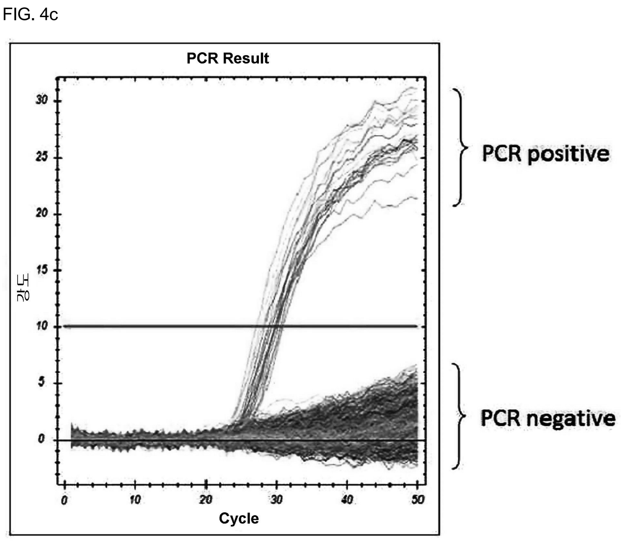

Referring again to FIGS. 1 and 2, the CMOS photosensor array 150 is disposed below the well array 140, 140' to capture the PCR reaction products in the plurality of wells of the well array 140, 140' in real time. Specifically, the CMOS photosensor array 150 is disposed to correspond to a substrate hole formed in the PCB 160 and receives light emitted from the well array 140, 140' to measure the products of the PCR reaction performed in the PCR device. For example, when the well array 140' is formed of a plurality of microwells, the measurement may be performed as illustrated in FIG. 4B. The measured PCR reaction products may be analyzed in a manner such as the graph shown on the right side of FIG. 4A or the graph shown in FIG. 4C.

FIG. 4A is a diagram showing the results of multiplex real-time PCR. The conceptual diagram on the left side of FIG. 4A represents the well array 140 according to the embodiment shown in FIG. 3A, and the graph on the right side shows the real-time fluorescence measurement values for each of the plurality of wells included in the well array 140.

FIGS. 4B and 4C show the results of digital real-time PCR. In particular, FIG. 4B is a photograph showing PCR positive/negative results in the well array 140' according to the embodiment shown in FIG. 3B, and FIG. 4C is a graph for detecting fluorescence in real time in each microwell to determine whether the corresponding well is positive or negative.

Referring again to FIGS. 1 and 2, the upper surface of the CMOS photosensor array 150 may be coated with a thin layer of material such as PDMS to form the bottoms of the plurality of wells after sealing.

The PCB 160 accommodates the CMOS image sensor 150. A vent hole 152 for vacuum processing may be formed in the PCB 160 so that liquid samples introduced through the inlet may be rapidly supplied to the well array 140, 140'. The vent hole 152 is in communication with the space between the CMOS photosensor array 150 and the well array 140, 140'.

In this embodiment, the PCR cartridge may further include a heater 170 for thermal cycling. As used herein, thermal cycling may include the step of using a thermal cycler, isothermal amplification, thermal convention, infrared mediated thermal cycling, or helicase dependent amplification. In some embodiments, the chip may be integrated with a built-in heating element. In various embodiments, the chip may also be integrated with semiconductors. Detection of targets according to various embodiments may include fluorescence detection, positive or negative ion detection, acidity (pH) detection, voltage detection, or current detection, either individually or in combination, but is not limited thereto.

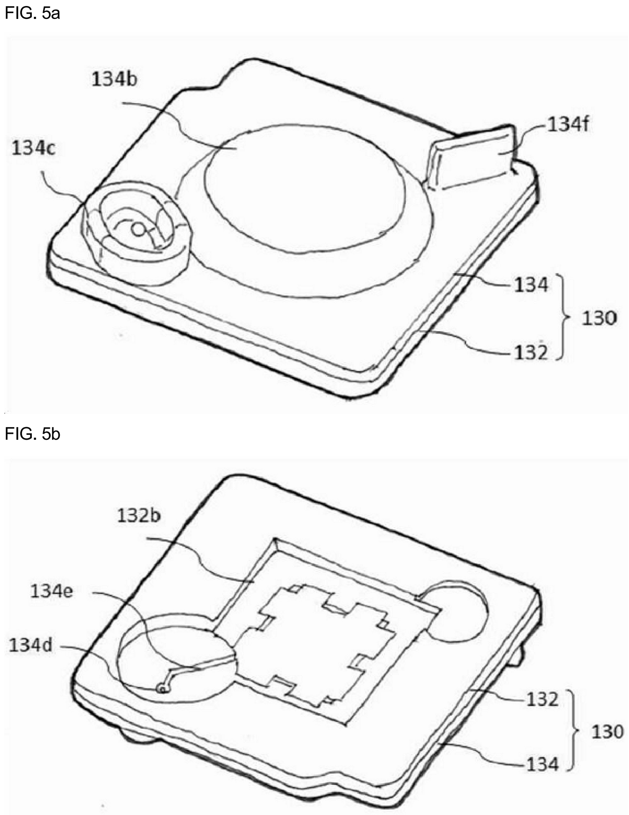

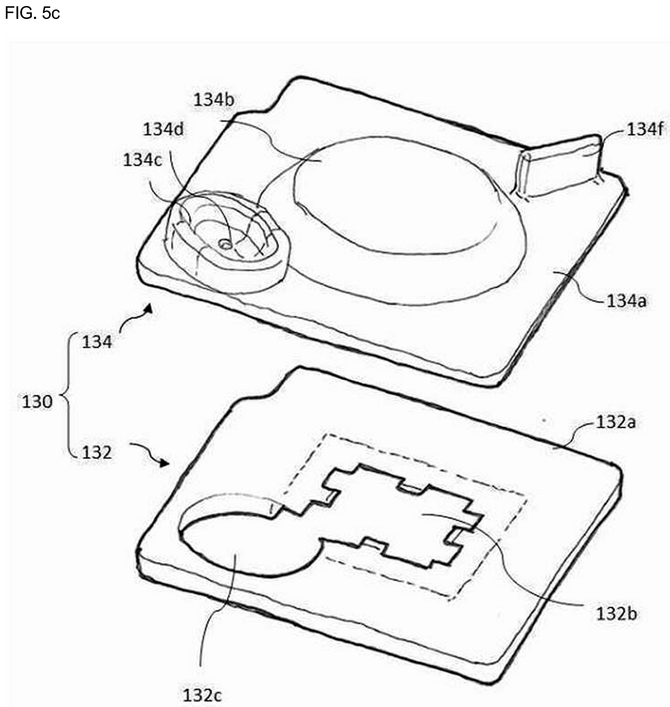

FIG. 5A is a front perspective view schematically illustrating the microfluidic chamber 130 illustrated in FIG. 2, FIG. 5B is a rear perspective view schematically illustrating the microfluidic chamber 130 illustrated in FIG. 2, and FIG. 5C is an exploded perspective view of the microfluidic chamber 130 illustrated in FIG. 2.

Referring to FIG. 2 to FIG. 5C, the microfluidic chamber 130 according to the present embodiment includes a base member 132 of a rectangular shape and a top member 134 of a rectangular shape disposed on the base member 132. In the present embodiment, the base member 132 and the top member 134 are shown as being physically separated, but these members may be integrally formed.

The base member 132 includes a first flat portion 132a of a rectangular shape, a support hole 132b of a rectangular shape formed in the center region of the first flat portion 132a, and a flat hole 132c of a circular shape formed in the corner region of the first flat portion 132a. Guide protrusions protruding toward the center region may be formed in the support hole 132b to define a rectangular sawtooth shape.

The top member 134 includes a second flat portion 134a of a rectangular shape, a membrane switch 134b of a dish shape, and an inlet portion 134c of a closed loop shape. The second flat portion 134a is in close contact with the upper surface of the first flat portion 132a. The membrane switch 134b is disposed in the central region of the second flat portion 134a and protrudes upward. The lower region of the membrane switch 134b corresponds to the support hole 132b of the base member 132. Accordingly, a recessed space is formed at the bottom of the microfluidic chamber 130. The CMOS image sensor 150 and the well array 140, 140' may be accommodated in the recessed space.

The inlet portion 134c has a fence shape and protrudes upward at one side of the membrane switch 134b to prevent the liquid samples from flowing out to the outside. An inlet 134d is formed in the central region of the inlet portion 134c in a direction perpendicular to the base member 132.

A microchannel 134e is formed in a region connecting the inlet 134d of the inlet portion 134c and the bottom edge region of the membrane switch 134b. The liquid sample introduced into the inlet 134d of the inlet portion 134c reaches the bottom edge region of the membrane switch 134b through the microchannel 134e.

The top member 134 may further include a grip portion 134f protruding upward from the observer's perspective on the opposite side of the membrane switch 134b. The grip portion 134f is disposed to face the inlet portion 134c with respect to the membrane switch 134b. The height of the grip portion 134f may be higher than the height of the inlet portion 134c. The height of the inlet portion 134c and the height of the membrane switch 134b are the same.

As described above, in the microfluidic chamber 130, the inlet 134d and the microchannel 134e are connected to each other. When the liquid sample is introduced through the inlet 134d, the liquid sample falls through the microchannel 134e into the space formed below the membrane switch 134b. Furthermore, when the membrane switch 134b is depressed, the liquid sample may completely fill each of the plurality of wells of the well array 140, 140' that is exposed through the microchannel 134e.

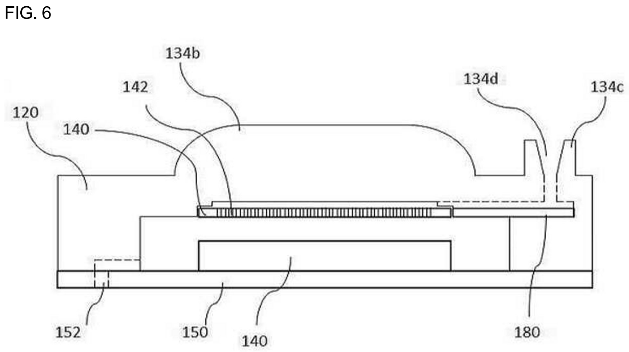

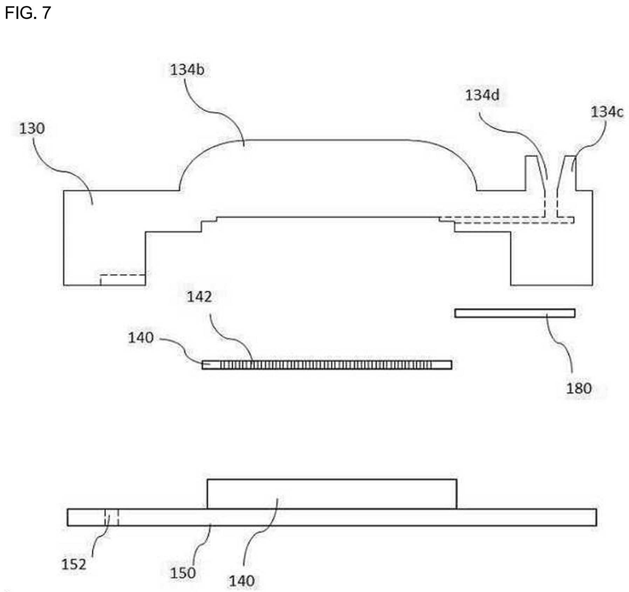

FIG. 6 is a cross-sectional view schematically illustrating the PCR module illustrated in FIG. 2. FIG. 7 is an exploded cross-sectional view schematically illustrating the PCR module illustrated in FIG. 6.

Referring to FIGS. 2 to 7, a PCR module according to an embodiment of the present invention includes a microfluidic chamber 130, a well array 140, 140', a CMOS photosensor array 150, and a PCB 150.

A recessed space is formed in the bottom region of the microfluidic chamber 130 to accommodate the CMOS photosensor array 150 and the well array 140, 140' disposed on the PCB 150. Since the microfluidic chamber 130 has been described in FIGS. 5A to 5C, a description thereof will be omitted.

The well array 140, 140' is attached to the lower surface of the microfluidic chamber 130. The well array 140, 140' is disposed on the CMOS photosensor array 150 and inserted into a substrate hole formed in the PCB 150. The well array 140, 140' includes a plurality of wells 142. The shape, size and number of the plurality of wells 142 may vary. Each of the plurality of wells 142 may contain an analytical sample such as a powder sample or a liquid sample. The analytical sample is a specific component for biological material analysis. That is, the analytical sample refers to a component for quantitative or qualitative analysis of a specific biological substance, such as a protein, DNA, or RNA, and includes, for example, a primer, a probe, an antibody, an aptamer, a DNA or RNA polymerase. In particular, it refers to a component necessary for a performing real-time polymerase chain reaction, isothermal enzymatic reaction, or ligase chain reaction (LCR).

The CMOS photosensor array 150 is disposed below the well array 140, 140' to capture images of the products of the PCR reaction performed in the plurality of wells 142 of the well array 140, 140' in real time. The CMOS photosensor array 150 is inserted into a substrate hole formed in the PCB 150. The CMOS photosensor array 150 receives the emitted light and captures images of the products of the PCR reaction performed in the PCR device in real time. That is, the CMOS photosensor array 150 detects fluorescence (emission light) generated from a plurality of probes by excitation light. The detection of the fluorescence may be performed by a time-division method or a wavelength-division separation method.

In the case of the time-division method, as the fluorescent substance emits light in response to excitation light, a fluorescence sensor array or a single sensor constituting the array detects the emitted light passing through an emission filter and detects the fluorescence by obtaining the time constant of the detected emitted light.

In the case of the wavelength-division method, as the fluorescent substance emits light in response to excitation light, a fluorescence sensor array or a single sensor constituting the array detects the emitted light passing through an emission filter and detects the fluorescence through spectral analysis of the detected emitted light.

The PCB 150 is disposed below the microfluidic chamber 130 to receive the CMOS photosensor array 150 mounted thereon. The PCB 150 is disposed in contact with the bottom edge region of the microfluidic chamber 130. A vent hole 152 is formed in a portion of the PCB 150 in contact with the bottom edge region of the microfluidic chamber 130. The vent hole 152 is connected to the space between the CMOS photosensor array 150 and the well array 140, 140'.

When a liquid sample is introduced into the inlet 134d, air is pumped through a vacuum device (not shown) connected to the vent hole 152. As the air is pumped, the liquid sample introduced into the inlet 134d is delivered to a partial region of the well array 140, 140', an upper region of the partial region, and a lower region of the partial region via the microchannel 134e formed in the microfluidic chamber 130.

In the present embodiment, the PCR module may further includes a sticker 180 that forms a bottom of the microchannel 134e formed in the microfluidic chamber 130. As the sticker 180 is attached to the microchannel 134e, the liquid sample introduced into the microchannel 134e may be supplied to the well array 140, 140' without being leaked to other areas. The thickness of the sticker 180 may be equal to the thickness of the well array 140, 140'.

In the present embodiment, the PCR module may further include a light providing part (not shown) configured to irradiate excitation light toward a probe contained in each of the plurality of wells 142 of the well array 140, 140'. In one embodiment, the light providing part may include a light source that emits light, such as a light emitting diode (LED) light source, a laser light source, or the like. The light emitted from a light source passes through or reflects the plurality of wells 142 of the well array 140, 140', and in this case, the CMOS photosensor array 150 may detect an optical signal generated by nucleic acid amplification.

In the present embodiment, the PCR module may further include an emission filter (not shown) configured to select light having a predetermined wavelength. The emission filter may be disposed on the CMOS photosensor array 150.

Next, a method of using a PCR cartridge according to an embodiment of the present invention will be described with reference to the drawings that sequentially illustrate the method. For convenience of explanation, an illustration of the upper case 110 (shown in FIG. 2) and the bottom case 120 (shown in FIG. 2) is omitted.

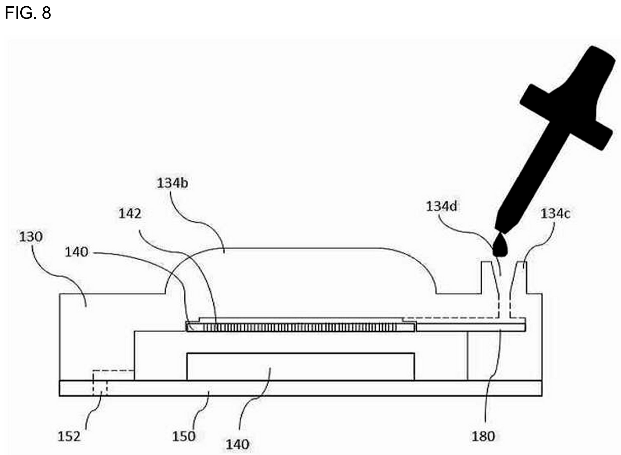

FIG. 8 is a cross-sectional view schematically illustrating a first step of the PCR process using the PCR module illustrated in FIG. 6.

Referring to FIG. 8, as a first step of the PCR process, an empty PCR module is disposed in a flat position.

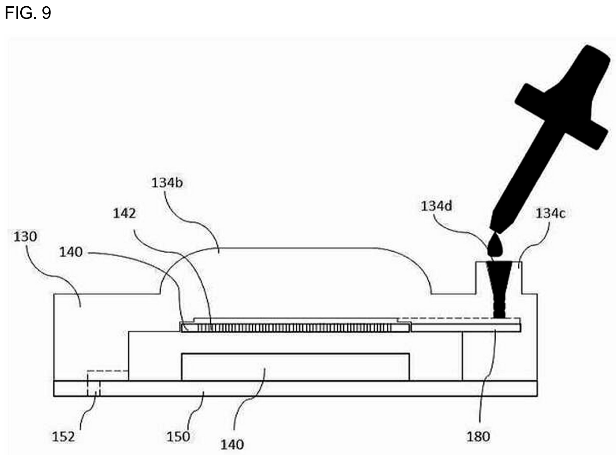

FIG. 9 is a cross-sectional view schematically illustrating a second step of the PCR process using the PCR module illustrated in FIG. 6.

Referring to FIG. 9, as a second step of the PCR process, a liquid sample is pipetted into the inlet 134d of the microfluidic chamber 130. Preferably, the amount of the liquid sample pipetted into the inlet 134d is sufficient to fill the space between the well array 140 and the microfluidic chamber 130, as well as the plurality of wells 142 of the well array 140, 140'. The liquid sample pipetted into the inlet 134d does not flow into the microchannel 134e due to the resistance of the air present in the microchannel 134e (shown in FIG. 3B).

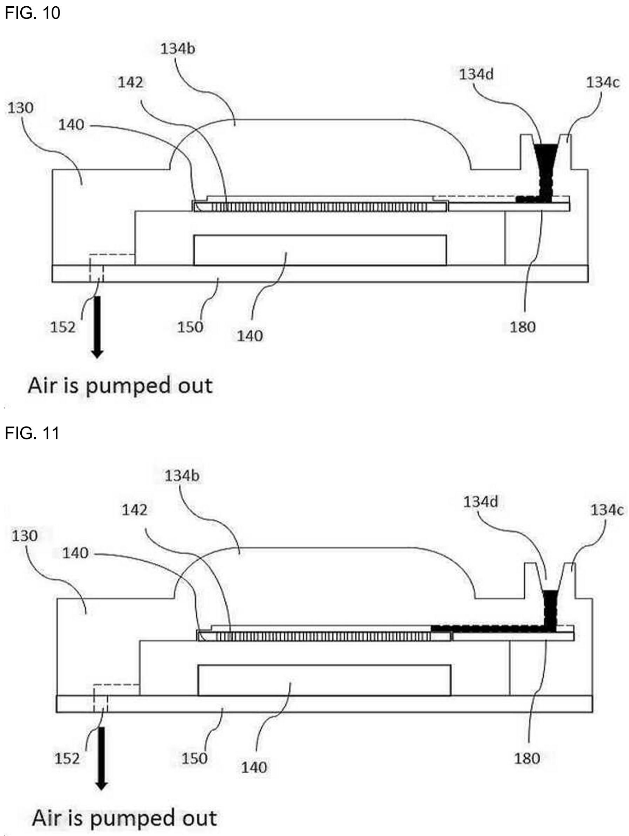

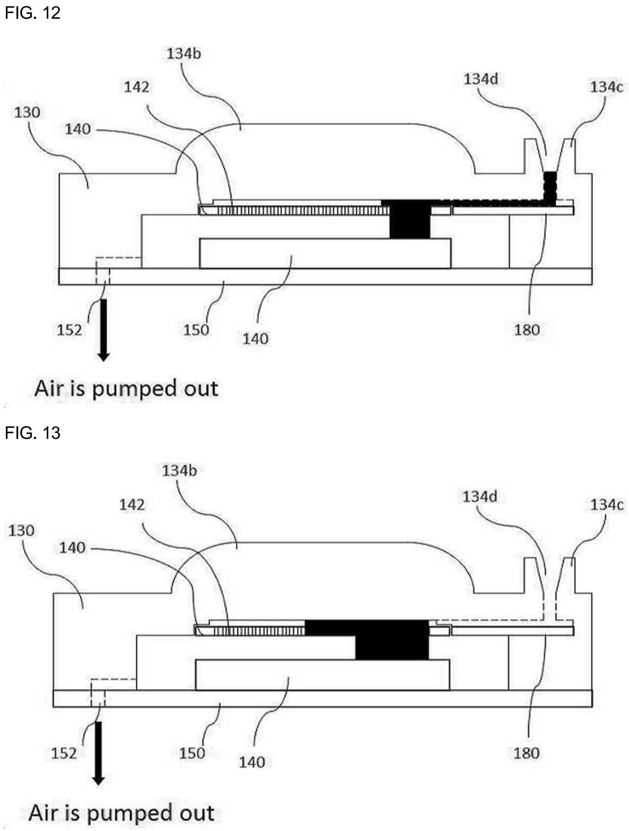

FIGS. 10, 11, 12 and 13 are cross-sectional views schematically illustrating a third step of the PCR process using the PCR module illustrated in FIG. 6.

Referring to FIGS. 10, 11, 12 and 13, as a third step of the PCR process, after the liquid sample is pipetted into the inlet 134e, a vacuum device (not shown) connected to the vent hole 152 is turned on.

Accordingly, air is pumped through the vent hole 152, and the liquid sample pipetted into the inlet 134e is delivered to the well array 140, 140' along the microchannel 134e. The liquid sample delivered to the well array 140, 140' reaches up to the CMOS photosensor array 150 through the plurality of wells 142 located near the terminal end of the microchannel 134e. This pumping of the vacuum device allows the liquid sample to be provided to the plurality of wells more quickly. Moreover, pumping of the vacuum device may more easily remove air that may be at the corner or edge regions of the well array.

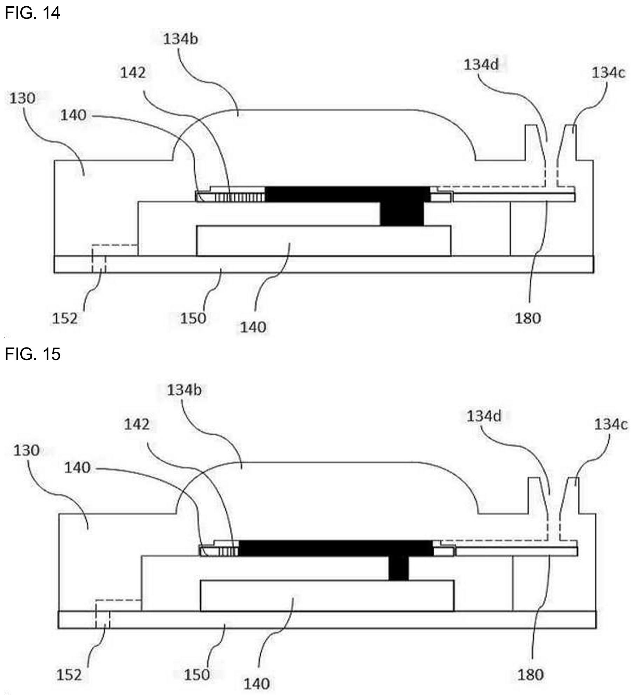

FIGS. 14, 15 and 16 are cross-sectional views schematically illustrating a fourth step of the PCR process using the PCR module illustrated in FIG. 6.

Referring to FIGS. 14, 15 and 16, as a fourth step of the PCR process, the vacuum device connected to the vent hole 152 is turned off. As the vacuum device is turned off, the liquid sample reaching the lower space of the well array 140, 140' is gradually drawn up to the upper space of the well array 140, 140' by the capillary force.

Accordingly, the liquid sample is filled in the space between the well array 140, 140' and the microfluidic chamber 130, as well as the plurality of wells 142 of the well array 140, 140'.

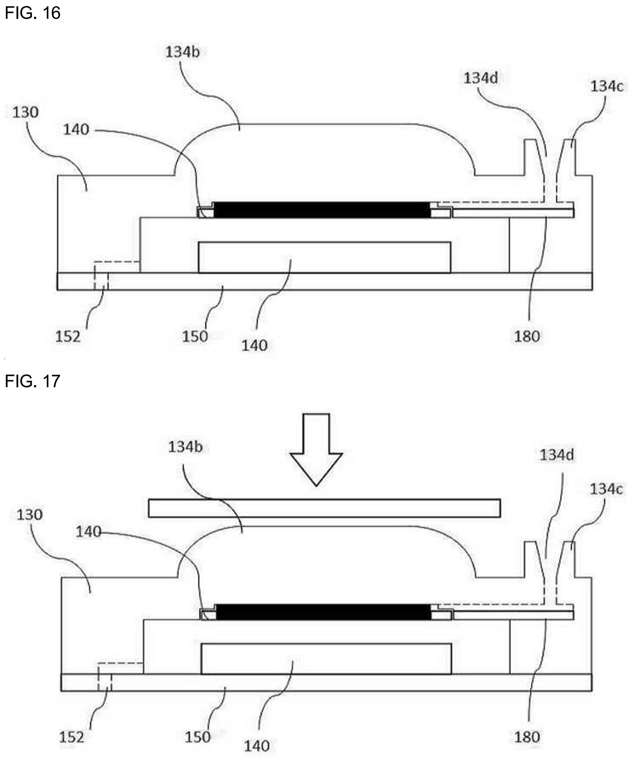

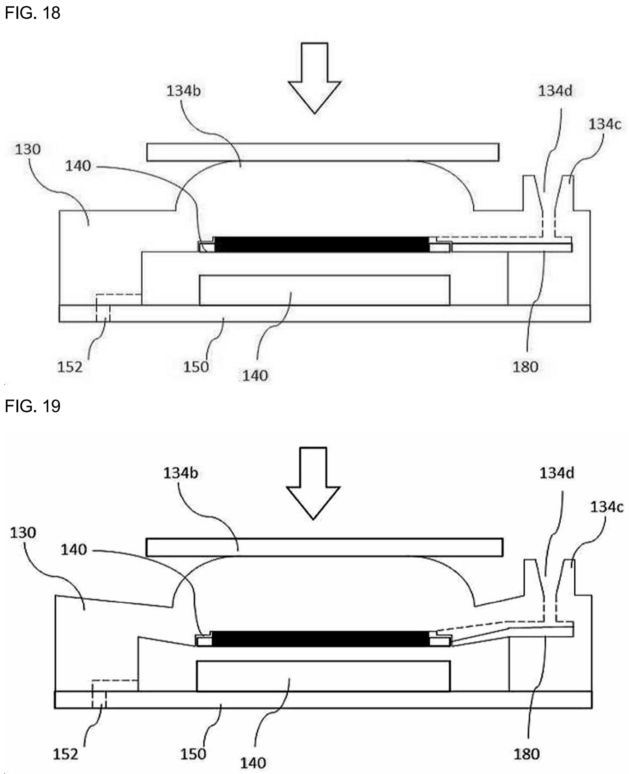

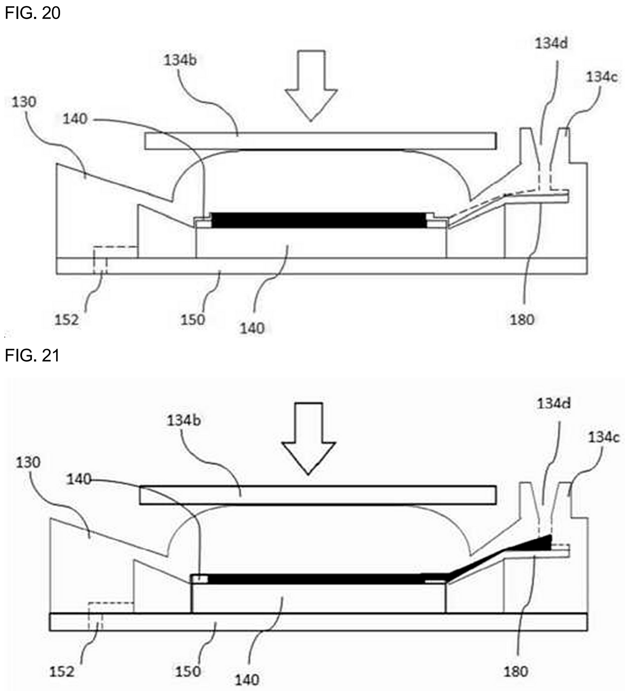

FIGS. 17, 18, 19, 20 and 21 are cross-sectional views schematically illustrating a fifth step of the PCR process using the PCR module illustrated in FIG. 6. In particular, the PCR process according to sealing is illustrated.

Referring to FIGS. 17, 18, 19, 20 and 21, as a fifth step of the PCR process, when the window member disposed in the central region of the upper case 110 is pressed in response to the user's pressing or the machine's pressing, the well array 140, 140' having a liquid sample filled in each of the plurality of wells 142 is pressed onto the CMOS photosensor array 150.

Accordingly, it is possible to prevent the formation of air pockets at the corner or edge regions of the well array 140, 140'. The air pockets expand or contract due to temperature changes during the PCR process, thereby causing errors in the test results. However, according to the present invention, when the PCR solution is moved through the pumping of the vacuum device and the PCR solution fills the reaction space, the formation of air pockets at the corners and edges of the well array, which serves as the reaction space, is prevented. Accordingly, it is possible to prevent errors in the PCR test results caused by such air pockets. In addition, since the well array is positioned on top of the CMOS photosensor array, the real-time PCR reaction can be measured.

As described above, according to the present invention, since the reagents are preloaded in the well array of the PCR module at the time of release, no separate procedure is required for setting the reagents, which significantly reduces the risk of contamination and eliminates the need for a separate procedure for preparing the test.

Moreover, even if the size of each of the plurality of wells in the well array, which serves as the reaction space, is very small and the number thereof is very large, it is possible to introduce the sample into each of the reaction spaces.

Furthermore, since the sample is introduced into the corner or edge regions of the well array, the formation of air pockets at the corner or edge regions of the well array, which serves as the reaction space, is prevented, and the reliability of the test results is improved.

A PCR method using the PCR cartridge of the present invention will be described below.

A method for diagnosing foot-and-mouth disease virus according to one aspect of the present invention may comprise:
a liquid sample injection step of injecting a liquid sample into an inlet to fill microwells;
a sensor contact step of bringing the microwells filled with the liquid sample into close contact with a CMOS photosensor;
a raw image acquisition step of acquiring raw images through the CMOS photosensor;
a fractionated image acquisition step of acquiring a plurality of fractionated images corresponding to each of the plurality of microwells facing the CMOS photosensor from the raw images;
a color parameter extraction step of extracting color parameters for each of a plurality of pixels assigned to each of the plurality of fractionated images;
a fraction determination value calculation step of calculating fraction determination values for each of the plurality of fractionated images based on an average value of the color parameters for the plurality of pixels; and
a final calculation step of calculating a final result value based on the plurality of fraction determination values,
wherein, in the raw image acquisition step, the raw image may be acquired at each predetermined time point.

The fractionated image acquisition step, the color parameter extraction step, the fraction determination value calculation step, and the final calculation step may be performed with respect to each raw image acquired at each predetermined time point, and the fraction determination value and the final result value may be acquired as time-series data.

The PCR analysis method of the present invention may update the final result value at each predetermined time point and output the updated value through a display device, thereby enabling the user to recognize the result value in real time. In the PCR analysis method of the present invention, the fraction determination value and the final result value may be output as time-series data, allowing various analysis results to be derived by analyzing the curves of the fraction determination values and the final result values over time (or cycles).

For example, in the sensor contact step, the liquid sample may be brought into close contact with the CMOS photosensor by creating a vacuum on the vent hole 152 side.

The raw image acquisition step may be performed after confirming that the plurality of microwells have been filled with the liquid sample through an empty well algorithm. The raw images acquired after the confirmation that the liquid sample has reached the wells may be used as valid data.

The fractionated image acquisition step may include: distinguishing the walls of the plurality of microwells from the plurality of microwells on the raw image; and acquiring, as the fractionated image, a portion of the raw image corresponding to each of the plurality of microwells.

Specifically, the walls between microwells may be distinguished through a contour algorithm.

In the color parameter extraction step, the color parameter may include at least one of brightness, saturation, hue, contrast, and color code. For example, the fractionated image may be processed into a complementary color image, and a contrast value may be assigned to each pixel to extract the color parameter. In another example, the saturation value of each pixel may be extracted as a color parameter. In yet another example, the brightness value, saturation value, hue value, and contrast value may be extracted from each pixel, and a value obtained by a linear function with independent weights assigned to each parameter may be extracted as a color parameter.

In the final result value calculation step, the degree of wetting of each of the plurality of microwells with respect to the liquid sample may be determined, and the final result value may be calculated only from the fraction determination values for the fractionated images of the microwells having a degree of wetting equal to or higher than a predetermined level.

In the final result value calculation step, the operational status of each of the plurality of microwells may be determined, and the final result value may be calculated only from the fraction determination values for the fractionated images of the microwells corresponding to a normal state among the plurality of microwells.

In the final result value calculation step, the fraction determination value may be output as time-series data as shown in FIG. 4B through a display device.

In the fraction determination value calculation step, the fraction determination value may be obtained as time-series data. For example, the fraction determination value may be obtained as a PCR amplification curve over time. That is, the fraction determination value may be obtained as a PCR amplification curve value.

In the final result value calculation step, the Ct value may be calculated from the fraction determination value (PCR amplification curve). The Ct value may refer to the threshold of cycle, which corresponds to the thermal cycle at which a fluorescence signal exceeding a certain intensity is first detected. In other words, the Ct value may provide information on the concentration (amount) of the target genetic material in the liquid sample.

In the final result value calculation step, the Ct values may be calculated for each concentration of the liquid sample. The Ct of each amplification curve may be determined for each concentration of the liquid sample, and their average Ct may be selected as a representative value. Linearity may be verified based on the average Ct for each concentration.

In conventional digital PCR analysis methods (e.g., endpoint digital PCR), the testing must be performed by diluting the sample until negative fractions are obtained. For example, if the minimum number of target genes required to yield all positive results in 20,000 partitioned digital PCR reactions is approximately 100,000, the dynamic range for target gene detection that can be determined in a single test would be from 0 to 100,000 copies.

However, the PCR analysis method of the present invention may enable high dynamic range analysis, as it provides real-time curve graphs for each fraction, as illustrated in FIG. 4C. For example, whereas conventional methods allow analysis within a dynamic range of up to 5 logs, the PCR analysis method of the present invention may allow analysis within a dynamic range of up to 9 logs.

According to another aspect of the present invention, there is provided a method for diagnosing foot-and-mouth disease virus of the present invention, the method comprising:
a liquid sample injection step of injecting a liquid sample into an inlet to fill a plurality of wells;
a sensor contact step of bringing the plurality of wells filled with the liquid sample into close contact with a CMOS photosensor;
a fluorescence signal detection step of detecting a fluorescence signal through the CMOS photosensor;
a Ct calculation step of calculating one or more cycle threshold (Ct) values for each of the plurality of wells from the detected fluorescence signals; and
a diagnosis step of diagnosing the presence and type of virus based on the cycle threshold values,
wherein the method diagnoses one or more of foot-and-mouth disease type O, foot-and-mouth disease type A, foot-and-mouth disease type Asial, foot-and-mouth disease type O/ME-SA/Ind-2001, foot-and-mouth disease type O/ME-SA/PanAsia, foot-and-mouth disease type O/SEA/Mya-98, foot-and-mouth disease type O/CATHAY, foot-and-mouth disease type A/ASIA/Sea-97, foot-and-mouth disease type A/ASIA/G-VII, vesicular stomatitis virus (VSV), swine vesicular disease virus (SVDV), and seneca valley virus (SVV).

In the injection step, the plurality of wells may each independently include primers and probes targeting one or more of foot-and-mouth disease type O, foot-and-mouth disease type A, foot-and-mouth disease type Asial, foot-and-mouth disease type O/ME-SA/Ind-2001, foot-and-mouth disease type O/ME-SA/PanAsia, foot-and-mouth disease type O/SEA/Mya-98, foot-and-mouth disease type O/CATHAY, foot-and-mouth disease type A/ASIA/Sea-97, foot-and-mouth disease type A/ASIA/G-VII, vesicular stomatitis virus (VSV), swine vesicular disease virus (SVDV), and seneca valley virus (SVV).

According to the method for diagnosing foot-and-mouth disease virus of the present invention, in the injection step, the plurality of wells may include a first fluorescent substance or a second fluorescent substance. According to one embodiment, the plurality of wells may be 2 or more, 6 or more, or 30 or less, but are not limited thereto. According to one embodiment, the first fluorescent substance and the second fluorescent substance may each independently be Biosearch Blue^{™}, FAM, TET, CAL Fluor^{®}, Dold 540, JOE, VIC, HEX, CAL Flour Orange 560, Quasar^{®} 570, Cy^{™} 3, NED, TAMRA, CAL Fluor Red 590, Cy 3.5, ROX, CAL Fluor Red 610, Texas Red^{®}, Cal Fluor Red 635, Pular^{®} 650, Cy 5, Quasar 670, Cy 5.5, Auasar 705, or CTBR Green.

According to one embodiment, the first fluorescent substance may be bound to a first probe targeting one of foot-and-mouth disease type O, foot-and-mouth disease type A, foot-and-mouth disease type Asial, foot-and-mouth disease type O/ME-SA/Ind-2001, foot-and-mouth disease type O/ME-SA/PanAsia, foot-and-mouth disease type O/SEA/Mya-98, foot-and-mouth disease type O/CATHAY, foot-and-mouth disease type A/ASIA/Sea-97, foot-and-mouth disease type A/ASIA/G-VII, and foot-and-mouth disease-like viruses including vesicular stomatitis virus (VSV), swine vesicular disease virus (SVDV), and seneca valley virus (SVV), and may be applied to one of the plurality of wells.

Moreover, the second fluorescent substance may be bound to a second probe targeting one of foot-and-mouth disease type O, foot-and-mouth disease type A, foot-and-mouth disease type Asial, foot-and-mouth disease type O/ME-SA/Ind-2001, foot-and-mouth disease type O/ME-SA/PanAsia, foot-and-mouth disease type O/SEA/Mya-98, foot-and-mouth disease type O/CATHAY, foot-and-mouth disease type A/ASIA/Sea-97, foot-and-mouth disease type A/ASIA/G-VII, and foot-and-mouth disease-like viruses including vesicular stomatitis virus (VSV), swine vesicular disease virus (SVDV), and seneca valley virus (SVV), and may be applied to one of the plurality of wells.

According to the method for diagnosing foot-and-mouth disease virus of the present invention, in the injection step, at least one first probe and at least one second probe may be applied together to one of the plurality of wells. Specifically, for example, a single well may include a first probe conjugated with a first fluorescent substance or a second probe conjugated with a second fluorescent substance. Furthermore, for example, a single well may include a first probe conjugated with a first fluorescent substance and a second probe conjugated with a second fluorescent substance. In addition, for example, at least one first probe and at least one second probe may be applied together to one of the plurality of wells.

If a single well includes both a first probe and a second probe, the first probe and the second probe may be distributed in separate regions within the well. Alternatively, the first probe and the second probe may be distributed together within the well.

According to still another aspect of the present invention, there is provided a PCR analysis device used in the method for diagnosing foot-and-mouth disease virus of the present invention, the PCR analysis device comprising:
a foot-and-mouth disease test cartridge including an in vitro diagnostic chip therein;
a foot-and-mouth disease test analysis module in which the foot-and-mouth disease test cartridge is mounted, the foot-and-mouth disease test analysis module being configured to receive an image or fluorescence signal from the foot-and-mouth disease test cartridge and to calculate a fraction determination value or a Ct value; and
a main server configured to receive the fraction determination value via a communication unit of the foot-and-mouth disease test analysis module.

According to the system of the present invention, the foot-and-mouth disease test analysis module may include a battery that supplies power and a communication unit that is communicatively connected to the main server.

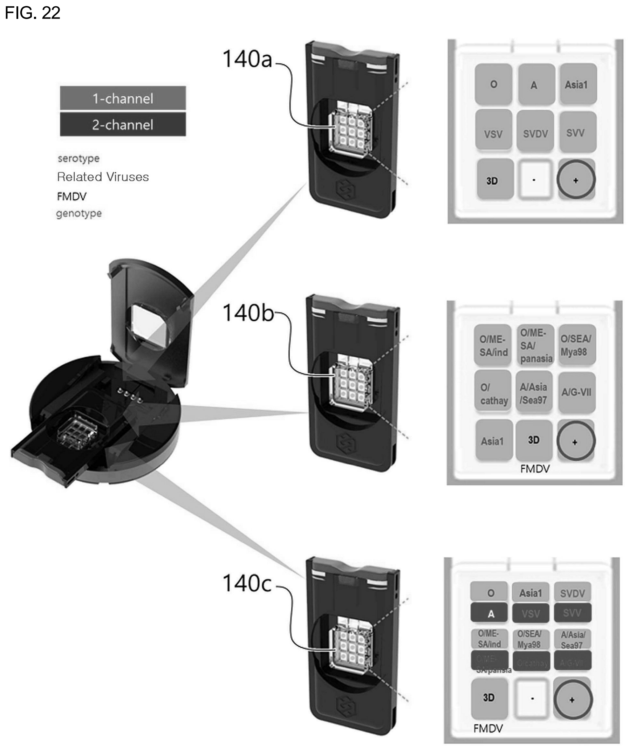

FIG. 22 is a photograph showing a PCR cartridge of the present invention. As shown in FIG. 22, depending on the target viruses to be diagnosed, a plurality of well arrays each containing different primers and probes may be provided such that the plurality of well arrays 140a, 140b and 140c can be detachably mounted in a housing composed of an upper case 110 and a bottom case 120. In other words, the plurality of well arrays 140a, 140b and 140c, which are provided in a modular form for a single housing, may be sequentially replaced to perform the analysis.

For example, the plurality of well arrays 140a, 140b and 140c may be provided as a serotype module 140a, a genotype module 140b, and a combined serotype/genotype module 140c.

According to another embodiment of the present invention, multiplex target analysis may be performed, as shown in the schematic diagram of FIG. 22. Foot-and-mouth disease virus is classified into seven serotypes (O, A, Asial, SAT1, SAT2, SAT3, and C). The cartridge of the present invention may comprise: a serotype module 140a configured to distinguish serotypes O and A, which are primarily detected in Korea, as well as Asia1 and related viruses (VSV, SVDV, and SVV) found in neighboring countries; a genotype module 140b configured to distinguish O/ME-SA/Ind-2001, O/ME-SA/PanAsia, O/SEA/Mya98, O/cathy, A/ASIA/Sea97, Asial, and A/ASIA/G-VII, and a combined serotype/genotype module 140c configured to comprehensively distinguish serotypes, genotypes, and related viruses in a single test.

As shown in FIG. 22, the primers and probes may be accommodated in each of the plurality of wells of the well array for single-channel detection using a single fluorescent substance (emitting a single fluorescence color), as in the serotype module 140a and the genotype module 140b. Alternatively, the primers and probes may be accommodated in each of the plurality of wells of the well array for multichannel detection using two or more fluorescent substances (emitting two or more fluorescence colors), as in the combined serotype/genotype module 140c.

The cartridge of the present invention illustrated in FIG. 22 may be applied to, for example, the devices disclosed in Korean Patent Application Nos. 10-2022-0041457 and 10-2020-0044341.

With the above-described features, the present invention provides a method for diagnosing foot-and-mouth disease virus, which can measure real-time reactions while preventing the formation of air pockets at the corner or edge regions of the reaction space. Accordingly, rapid and accurate diagnosis and analysis of foot-and-mouth disease virus can be achieved.

Next, embodiments of the present invention will be described in detail so that those skilled in the art to which the present invention pertains may easily carry out the invention. However, the present invention may be embodied in various different forms and is not limited to the embodiments described herein.

### EXAMPLES

In the following example, AccuPower^{®} FMDV Real-Time RT-PCR MasterMix Kit (Bioneer) was used.

### Example 1

Viral culture fluids of FMDV serotypes O, A, and Asia1 were prepared at concentrations ranging from 10⁶ TCID₅₀/mL to 1 TCID₅₀/mL, and RNA was extracted from the FMDV samples. AccuPower^{®} FMDV Real-Time RT-PCR MasterMix Kit (Bioneer) was used for the detection of each virus. A mixture was prepared to have a total volume of 30 µL per reaction. Specifically, 5 µL of RNA sample and 25 µL of FMDV MasterMix (AccuPower^{®} FMDV Real-Time RT-PCR MasterMix Kit, Bioneer Co.), which contains primers, probes, enzymes, and buffer, were added to a tube to prepare a mixture having a total volume of 30 µL. The prepared mixture (30 µL) was injected into the inlet of the cartridge of the present invention. PCR was performed under the following conditions: reverse transcription to convert RNA into cDNA at 45 °C for 30 minutes for 1 cycle, followed by denaturation of the cDNA at 95 °C for 5 minutes for 1 cycle.

Each well is completely isolated after the PCR mixture is injected, allowing the PCR reaction to occur independently in each well, and two fluorescence channels detect the signals. Since each well has a fixed volume, it is configured to eliminate user-to-user variation that may occur during the experimental process. Using a semiconductor biosensor PCR technique, the light detection and thermal regulation semiconductors of the chip case precisely perform the PCR process and sensitively detect fluorescence signals, allowing the software to analyze the results. The limit of detection (LOD) of the diagnostic kit was determined in accordance with the Clinical and Laboratory Standards Institute (CLSI) EP-17 guideline.

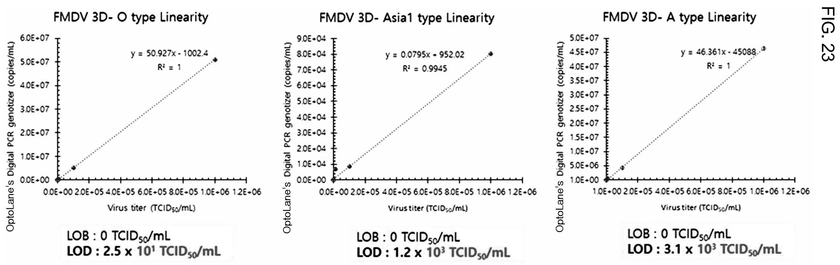

The virus titers (TCID₅₀) of each virus according to Example 1 are shown in FIG. 23.

### Experimental Example 1

To verify the effectiveness of the present invention, real-time PCT analysis was performed using the conventional AccuPower^{®} FMDV Real-Time RT-PCR MasterMix Kit (Bioneer) and the CFX96 Touch Deep Well Real-Time PCR Detection System (Bio-Rad), and the number of DNA copies for each virus was compared.

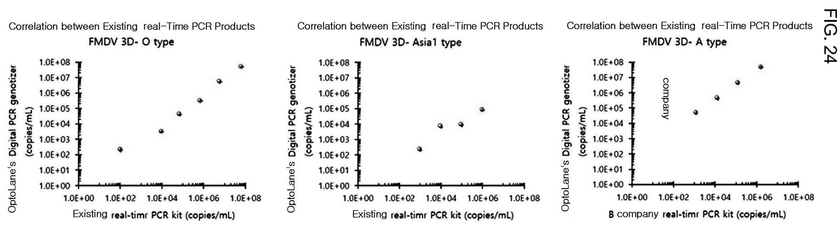

FIG. 24 is a graph illustrating the degree of consistency between the results of the conventional real-time PCR and those of Example 1.

Although certain embodiments of the present invention have been described in detail above, it will be apparent to those skilled in the art to which the present invention pertains that such detailed descriptions are merely exemplary of preferred embodiments and should not be construed as limiting the scope of the present invention. Those skilled in the art will be able to make various applications and modifications within the scope of the present invention based on the above description. Therefore, the true scope of the present invention is defined by the appended claims and their equivalents.

## Claims

1. A method for diagnosing foot-and-mouth disease virus, the method comprising:
a liquid sample injection step of injecting a liquid sample into an inlet to fill a plurality of wells;
a sensor contact step of bringing the plurality of wells filled with the liquid sample into close contact with a CMOS photosensor;
a fluorescence signal detection step of detecting a fluorescence signal through the CMOS photosensor;
a Ct calculation step of calculating one or more cycle threshold (Ct) values for each of the plurality of wells from the detected fluorescence signals; and
a diagnosis step of diagnosing the presence and type of virus based on the cycle threshold values,
wherein the method diagnoses one or more of foot-and-mouth disease type O, foot-and-mouth disease type A, foot-and-mouth disease type Asial, foot-and-mouth disease type O/ME-SA/Ind-2001, foot-and-mouth disease type O/ME-SA/PanAsia, foot-and-mouth disease type O/SEA/Mya-98, foot-and-mouth disease type O/CATHAY, foot-and-mouth disease type A/ASIA/Sea-97, foot-and-mouth disease type A/ASIA/G-VII, vesicular stomatitis virus (VSV), swine vesicular disease virus (SVDV), and seneca valley virus (SVV).

2. The method for diagnosing foot-and-mouth disease virus according to claim 1, wherein, in the injection step, the plurality of wells each independently comprises primers and probes targeting one or more of foot-and-mouth disease type O, foot-and-mouth disease type A, foot-and-mouth disease type Asial, foot-and-mouth disease type O/ME-SA/Ind-2001, foot-and-mouth disease type O/ME-SA/PanAsia, foot-and-mouth disease type O/SEA/Mya-98, foot-and-mouth disease type O/CATHAY, foot-and-mouth disease type A/ASIA/Sea-97, foot-and-mouth disease type A/ASIA/G-VII, vesicular stomatitis virus (VSV), swine vesicular disease virus (SVDV), and seneca valley virus (SVV).

3. The method for diagnosing foot-and-mouth disease virus according to claim 2, wherein, in the injection step, the plurality of wells comprise a first fluorescent substance or a second fluorescent substance.

4. The method for diagnosing foot-and-mouth disease virus according to claim 3, wherein the first fluorescent substance is bound to a first probe targeting one of foot-and-mouth disease type O, foot-and-mouth disease type A, foot-and-mouth disease type Asial, foot-and-mouth disease type O/ME-SA/Ind-2001, foot-and-mouth disease type O/ME-SA/PanAsia, foot-and-mouth disease type O/SEA/Mya-98, foot-and-mouth disease type O/CATHAY, foot-and-mouth disease type A/ASIA/Sea-97, foot-and-mouth disease type A/ASIA/G-VII, and foot-and-mouth disease-like viruses including vesicular stomatitis virus (VSV), swine vesicular disease virus (SVDV), and seneca valley virus (SVV), and is applied to one of the plurality of wells, and
wherein the second fluorescent substance is bound to a second probe targeting one of foot-and-mouth disease type O, foot-and-mouth disease type A, foot-and-mouth disease type Asial, foot-and-mouth disease type O/ME-SA/Ind-2001, foot-and-mouth disease type O/ME-SA/PanAsia, foot-and-mouth disease type O/SEA/Mya-98, foot-and-mouth disease type O/CATHAY, foot-and-mouth disease type A/ASIA/Sea-97, foot-and-mouth disease type A/ASIA/G-VII, and foot-and-mouth disease-like viruses including vesicular stomatitis virus (VSV), swine vesicular disease virus (SVDV), and seneca valley virus (SVV), and is applied to one of the plurality of wells.

5. The method for diagnosing foot-and-mouth disease virus according to claim 4, wherein, in the injection step, at least one first probe and at least one second probe are applied together to one of the plurality of wells.

6. A PCR analysis method using a PCR cartridge, the cartridge comprising: a microfluidic chamber including an inlet formed for introduction of a liquid sample and capable of being produced by injection molding; a well array including a plurality of microwells through which upper and lower portions are perforated, the well array being attached to a lower surface of the microfluidic chamber; a CMOS photosensor array disposed below the well array and configured to capture real-time reaction images of samples filled in the microwells of the well array; and a PCB having a vent formed for vacuum processing of a microchannel that is provided in the microfluidic chamber, a space that is created between the well array and the microfluidic chamber, and the microwells that is formed in the well array, as the liquid sample is introduced through the inlet,
the method comprising:
a liquid sample injection step of injecting the liquid sample into the inlet to fill the microwells;
a sensor contact step of bringing the microwells filled with the liquid sample into close contact with a CMOS photosensor;
a raw image acquisition step of acquiring raw images through the CMOS photosensor;
a fractionated image acquisition step of acquiring a plurality of fractionated images corresponding to each of the plurality of microwells facing the CMOS photosensor from the raw images;
a color parameter extraction step of extracting color parameters for each of a plurality of pixels assigned to each of the plurality of fractionated images;
a fraction determination value calculation step of calculating fraction determination values for each of the plurality of fractionated images based on an average value of the color parameters for the plurality of pixels; and
a final calculation step of calculating a final result value based on the plurality of fraction determination values,
wherein, in the raw image acquisition step, the raw image is acquired at each predetermined time point, and
wherein the PCR analysis method diagnoses one or more of foot-and-mouth disease type O, foot-and-mouth disease type A, foot-and-mouth disease type Asial, foot-and-mouth disease type O/ME-SA/Ind-2001, foot-and-mouth disease type O/ME-SA/PanAsia, foot-and-mouth disease type O/SEA/Mya-98, foot-and-mouth disease type O/CATHAY, foot-and-mouth disease type A/ASIA/Sea-97, foot-and-mouth disease type A/ASIA/G-VII, vesicular stomatitis virus (VSV), swine vesicular disease virus (SVDV), and seneca valley virus (SVV).

7. The method for diagnosing foot-and-mouth disease virus according to claim 6, wherein the fractionated image acquisition step, the color parameter extraction step, the fraction determination value calculation step, and the final result value calculation step are performed with respect to each raw image acquired at each predetermined time point, and
wherein the fraction determination value and the final result value are obtained as time-series data.

8. The method for diagnosing foot-and-mouth disease virus according to claim 6, wherein the raw image acquisition step is performed after confirming that the plurality of microwells have been filled with the liquid sample.

9. The method for diagnosing foot-and-mouth disease virus according to claim 6, wherein the fractionated image acquisition step comprises:
distinguishing the walls of the plurality of microwells from the plurality of microwells on the raw image; and
acquiring, as the fractionated image, a portion of the raw image corresponding to each of the plurality of microwells.

10. The method for diagnosing foot-and-mouth disease virus according to claim 6, wherein, in the color parameter extraction step, the color parameter comprises at least one of brightness, saturation, hue, contrast, and color code.

11. The method for diagnosing foot-and-mouth disease virus according to claim 6, wherein, in the final result value calculation step,
the degree of wetting of each of the plurality of microwells with respect to the liquid sample is determined, and
the final result value is calculated only from the fraction determination values for the fractionated images of the microwells having a degree of wetting equal to or higher than a predetermined level.

12. The method for diagnosing foot-and-mouth disease virus according to claim 6, wherein, in the final result value calculation step,
the operational status of each of the plurality of microwells is determined, and
the final result value is calculated only from the fraction determination values for the fractionated images of the microwells corresponding to a normal state among the plurality of microwells.

13. A foot-and-mouth disease virus test system for performing foot-and-mouth disease virus diagnosis by the method according to claim 1 or 6, the system comprising:
a foot-and-mouth disease test cartridge including an in vitro diagnostic chip therein;
a foot-and-mouth disease test analysis module in which the foot-and-mouth disease test cartridge is mounted, the foot-and-mouth disease test analysis module being configured to receive an image or fluorescence signal from the foot-and-mouth disease test cartridge and to calculate a fraction determination value or a Ct value; and
a main server configured to receive the fraction determination value via a communication unit of the foot-and-mouth disease test analysis module.

14. The foot-and-mouth disease virus test system according to claim 13, wherein the foot-and-mouth disease test analysis module comprises a battery that supplies power and a communication unit that is communicatively connected to the main server.
